## Europäisches Patentamt
## European Patent Office
### Office européen des brevets

(19)

(11) Publication number: **0 096 942**
**B1**

(12)

# EUROPEAN PATENT SPECIFICATION

(45) Date of publication of patent specification: **19.03.86**

(21) Application number: **83200868.4**

(22) Date of filing: **14.06.83**

(51) Int. Cl.⁴: **A 61 K 31/65,** A 61 K 9/08,
A 61 K 47/00, C 07 C 103/19

(54) Oxytetracycline composition and process for preparing an oxytetracycline composition.

(30) Priority: **15.06.82 NL 8202423**

(43) Date of publication of application:
**28.12.83 Bulletin 83/52**

(45) Publication of the grant of the patent:
**19.03.86 Bulletin 86/12**

(84) Designated Contracting States:
**BE DE FR GB NL SE**

(56) References cited:
DE-B-1 053 736
GB-A-1 427 882
GB-A-1 592 053
GB-A-2 000 970
NL-A- 134 511
US-A-4 018 889
US-A-4 086 332

CHEMICAL ABSTRACTS, vol. 52, no. 2, 1958,
column 1552q, Columbus, Ohio, USA, e.H.
GANS et al.: "The solubility and complexing
properties of oxytetracycline. II. Interactions in
nonaqueous solution"
Repertorium Dierg. 1e EDITIE 1978/79,
intgevery De Toorts Haarlem - XIII
Chemoterapeutica 73

The file contains technical information
submitted after the application was filed and
not included in this specification

(73) Proprietor: **Aesculaap B.V.**
**P.O. Box 35**
**NL-5280 AA Boxtel (NL)**

(72) Inventor: **Pauw, Cornelis**
**P.O. Box 630**
**NL-1500 EP Zaandam (NL)**

(74) Representative: **van der Saag, Johannes**
**OCTROOIBUREAU VRIESENDORP & GAADE**
**P.O. Box 266**
**NL-2501 AW The Hague (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**Description**

The invention relates to an oxytetracycline composition which, on 100 parts by volume of the composition comprises 10—30 parts by weight of oxytetracycline in the form of a complex with alkaline earth metal ions, and to a process for preparing an oxytetracycline composition wherein a solution is formed of alkaline earth metal complexes of oxytetracycline in a mixture of water, water-miscible organic solvent and adjuvants with a pH between 6.0 and 9.5.

Such a composition and process are known from Dutch patent specification 141 086 which describes the preparation of oxytetracycline compositions which comprise 0.1—15 parts by weight oxytetracycline, in the form of an alkaline earth metal complex, per 100 vol. parts of solution (here and also further in the specification and in the claims the ratio of parts by weight and vol. parts is g:ml), by dissolving alkaline earth metal compounds of oxytetracycline in a mixture of water and of glycerol formal.

The average pH of said solutions is in practice between 8 and 9.

In Dutch patent application 7713761 a process is described for preparing oxytetracycline compositions which on average have a somewhat lower pH, wherein an aqueous solution is formed with at most 15% by weight of oxytetracycline, sufficient alkaline earth metal ions to form a complex with the oxytetracycline, 4—6% by weight of polyvinyl pyrrolidone as stabilizer and 15—40% by weight of an aliphatic amide as organic solvent and the pH is adjusted at 5.0—7.5.

With those compositions a solution of an alkaline earth metal complex of oxytetracycline is formed, since oxytetracycline base and the acid addition salts thereof per se are difficult to dissolve and moreover are not stable in aqueous solution.

All these known compositions and processes have in common, that the oxytetracycline content of the solutions is not more than 15 parts by weight per 100 vol. parts solution.

From U.S. patent specification 4 018 889 oxytetracycline compositions are known, with contents of oxytetracycline or pharmaceutically acceptable acid addition salts thereof, between 1 and 40% by weight, the solvent of which comprising a mixture of water and 10—50% by weight of 2-pyrrolidone, which compositions have as main indication, the application as parenteral injection solution, and as sub-indication, composition for topical use.

It was now found, that completely stable oxytetracycline compositions can be prepared, in which the oxytetracycline content can vary between 10 and 30 parts by weight oxytetracycline per 100 parts by volume of solution, which compositions, besides for normal parenteral use, are particularly suitable for intravenous and intraperitoneal use and moreover provide an extremely high oxytetracycline-blood serum level and very little tissue irritation, if with the preparation of the compositions, as organic solvent N-methylpyrrolidone is used and further in the composition a certain amount of polyvinylpyrrolidone is incorporated.

The invention now provides an oxytetracycline composition which, on 100 parts by volume of the composition, comprises 10—30 parts by weight of oxytetracycline in the form of a complex with alkaline earth metal ions, 30—60 parts by volume of water, 2.5—10 parts by weight of polyvinylpyrrolidone usual amounts of antioxidants, inorganic or organic bases in an amount sufficient to bring the pH of the composition at a value between 6.0 and 9.5, the balance being N-methylpyrrolidone.

In this composition the N-methylpyrrolidone contributes to the solubility of the oxytetracycline complex and the stability of the composition. The polyvinylpyrrolidone leads to less tissue irritation and to a better toleration of the composition on injection; indeed the polyvinylpyrrolidone gives some increase in the viscosity of the composition, but with the used amounts this increase in the viscosity is slight and is fully compensated by the improved toleration and decreased tissue irritation.

We remark that the use of concentrated solutions of N-methylpyrrolidone as solvent for pharmaceutical compositions is already generally described in Dutch patent specification 89788. However, oxytetracycline is not mentioned there and it could not be expected or predicted that N-methylpyrrolidone would allow the preparation of oxytetracycline compositions with a high concentration of oxytetracycline-alkaline earth metal complex, which have said favourable properties.

As remarked above, also according to Dutch patent application 7713761 a slight percentage of polyvinylpyrrolidone is used as stabilizer in the compositions described there.

In the process according to the invention, however polyvinylpyrrolidone is not used as stabilizer.

In Dutch patent specification 134 511 the preparation of oxytetracycline compositions for parenteral administration is described, wherein alkaline earth metal complexes of oxytetracycline are incorporated into a mixture of water and polyvinylpyrrolidone (PVP), the pH of which is adjusted at 8.0—9.5.

The compositions in question do not comprise a water-miscible organic solvent and comprise 7.5—25 parts by weight of PVP in 100 ml of the composition. It appeared however, that oxytetracycline compositions with 20% oxytetracycline, if they comprise PVP in amounts of more than 10 parts by weight in 100 ml of composition, have such a viscosity that they cannot be parenterally administered anymore.

It is surprising that the composition of the invention is not only suitable for normal parenteral use, but also for intravenous and intraperitoneal use; commercial compositions of the type known from U.S. patent specification 4 018 889 just have a contra-indication against intravenous administration.

Intraperitoneal injection is not mentioned in U.S. patent specification 4 018 889. This way of injecting has advantages in case of e.g. peritonitis; the oxytetracyline is then quickly at the place where it should be.

2

The preferred process according to the invention is characterized in that 10—30 parts by weight of oxytetracycline in the form of the base or an acid addition salt and sufficient alkaline earth metal ions to form a complex with the oxytetracycline 2.5—10 parts by weight of polyvinyl pyrrolidone and usual amounts of anti-oxidants are incorporated into 30—60 parts by volume of water, the pH is adjusted at a value between 6.0 and 9.5 by adding an inorganic or organic base and the mixture is made up to a volume of 100 parts by volume with N-methyl-pyrrolidone.

Preferably, according to the invention one prepares compositions which comprise 20—25 parts by weight of oxytetracycline per 100 parts by volume of solution; said compositions allow an especially favourable decrease in the amount to be administered for a certain dosage of oxytetracycline.

The alkaline earth metal complex of oxytetracycline is formed in situ in the process according to the invention, in that oxytetracycline in the form of the free base, in particular the dihydrate of the free base, or of an acid addition salt, in particular the hydrochloride and sufficient alkaline earth metal ions to form a complex with the oxytetracycline are incorporated into water.

The alkaline earth metal ions by which the complex is formed, are preferably magnesium ions, as with magnesium ions most stable complexes are formed.

As source of magnesium ions every magnesium compound can be used, e.g. magnesium chloride or magnesium oxide, but preferably magnesium oxide is used, as herewith no foreign anions are brought in the solution.

As antioxidants in the first place current antioxidants are used, such as sodium metabisulphite, sodium sulphite or sodium formaldehyde sulfoxylate; said antioxidants are incorporated into the solution in a small amount e.g. in an amount of 0.1—1.0 parts by weight on 100 parts by vol. of solution to promote the shelf life of the solution.

Suitable agents for adjusting the pH are physiologically innocuous inorganic or organic bases, such as sodium hydroxide, ammonia, monoethanolamine, ethylene diamine.

The polyvinylpyrrolidone which is incorporated into the compositions according to the invention has a molecular weight between 1000 and 60000. Preferably polyvinylpyrrolidone with a molecular weight between 2500 and 15000 is used, as this has the best tissue protecting activity.

Polyvinylpyrrolidone with such a molecular weight is also used in a 3.5—4.5% solution in water as substitute for blood plasma (Römp, Chemie, Lexikon, volume III, 53 Ed. Stuttgart 1962).

The compositions which are prepared by using said components have an excellent stability, are suitable for intravenous and intraperitoneal administration, give high oxytetracycline levels in the blood after intramuscular injection and less tissue irritation.

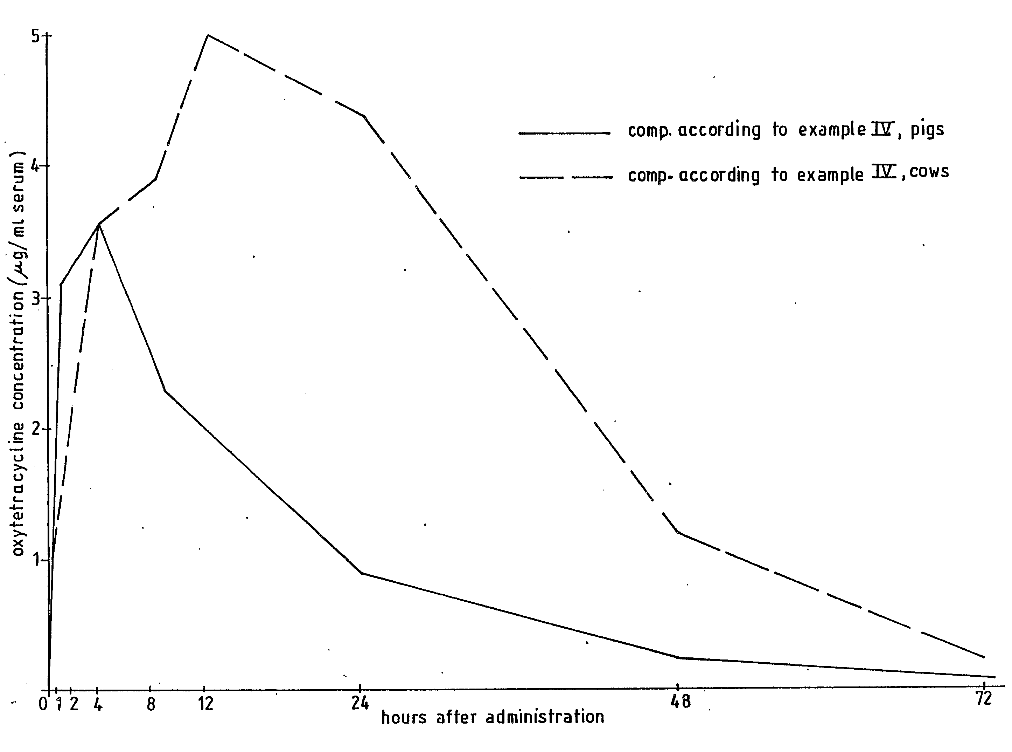

The invention is elucidated by the following examples and by figure 1.

Figure 1 is a graph, which shows on the ordinate the oxytetracycline level in µg/ml serum of pigs or cows, over a period of 72 hours (on the abscis) after an intra-muscular injection of 20 mg oxytetracycline/kg body weight.

The invention is elucidated by the following examples.

Example I

In 45 ml distilled, sterile and pyrogen-free water (water for injections) 0.5 g of sodium formaldehyde sulfoxylate were dissolved. Subsequently 1.7 g of magnesium oxide were added while stirring and stirring was continued until a homogeneous suspension was formed, whereafter 21.6 g of oxytetracycline dihydrate were added.

5 g of polyvinylpyrrolidone (PVP) of molecular weight 11500 were added and with monoethanolamine the pH of the mixture was brought at 8.5 (about 1.3 ml monoethanol amine); thereafter the mixture was made up with N-methylpyrrolidone up to a volume of 100 ml. This resulted in a stable solution with a pH of 8.5 and a viscosity of 30 cp, which comprised 200 mg oxytetracycline per ml. Said solution was sterilized by filtration and filled up in ampoules.

In order to dissolve the oxytetracycline faster, it is favourable to add first a portion of the total required amount of N-methyl-pyrrolidone to the water and thereafter to incorporate all other components into said mixture. This is elucidated in example II.

Example II

45 ml of distilled, sterile and pyrogen-free water were mixed with N-methylpyrrolidone up to a total volume of 80 ml. In said mixture 0.5 of sodium formaldehyde sulfoxilate were dissolved, whereafter during stirring 1.7 g magnesium oxide were added and stirring was continued until a homogenous suspension was obtained. 21.6 g of oxytetracycline-dihydrate and 5 g of PVP of molecular weight 11500 were added to said suspension, whereafter the pH of the mixture, was brought at 8.0 with monoethanolamine. After the mixture became clear the pH was brought at 8.5 with monoethanolamine and thereafter the mixture was made up with N-methylpyrrolidone to a volume of 100 ml.

Example III

The process of example I or II was repeated, while the oxytetracyline dihydrate was replaced by oxytetracycline hydrochloride, the PVP of molecular weight 11500 was replaced by a similar amount of PVP of molecular weight 2500 and the amounts of magnesium oxide and monoethanolamine were adapted.

3

The amounts of the components which were used and the properties of the compositions are summarized in the table. Also this solution was completely stable.

|  | Examples I and II | Example III |
| --- | --- | --- |
| oxytetracycline-dihydrate (g) | 21.6 | — |
| oxytetracycline HCl (g) | — | 22 |
| MgO (g) | 1.7 | 2.5 |
| water for injections (ml) | 45 | 45 |
| sodium formal dehydesulfoxylate (g) | 0.5 | 0.5 |
| PVP with MG 11500 | 5 | — |
| PVP with MG 2500 (G) | — | 5 |
| monoethanolamine (ml used) | until pH 8.5 (1.3) | until pH 8.5 (1.6) |
| N-methylpyrrolidone (ad 100 ml) | added | added |
| pH | 8.5 | 8.5 |
| viscosity (cp) | 30 | 30 |

Example IV

A number of test animals (cows, resp. pigs) were intramuscularly injected with the composition according to example I. The dosage was 20 mg oxytetracycline/kg body weight.

One worked as follows:

Test animals

10 pigs with an average weight of 25 kg; the pigs were fed with antibiotics-free feed during one month before the test;

5 yearlings with a weight estimated at 250 kg; the yearlings were stabled during the experiment.

Administration

The compositions were administered intramuscularly, in one injection; in the pigs behind the left ear, in the yearlings in the left part of the neck.

Tapping blood

The blood was tapped with the aid of a vacutainer; in the pigs from the a. carotis, in the yearlings from the v. jugularis. Blood samples were taken just before the injection (blank) and in the pigs 1, 4, 9, 24, 48 and 72 hours after injection; in the yearlings 1/2, 1, 2, 4, 8, 12, 24, 48 and 72 hours after injection.

After being tapped the blood was centrifuged as soon as possible and the serum was kept in a freezer at −20°C until the analysis. The oxytetracycline content was determined microbiologically with the aid of Bacillus cereus ver. mycoides ATCC 11778. The culture medium used was Standard Nähragar (Merck) with 0.2% $KH_2PO_4$; pH 6.0; the oxytetracycline standard was made with blank sera of two species of animals.

The results are indicated in tables A and B and are graphically shown in the figure.

### TABLE A
Oxytetracycline content in µg/ml in the serum of pigs

| Pig | Composition | Hours after treatment | | | | | | |
|---|---|---|---|---|---|---|---|---|
| | | 0 | 1 | 4 | 9 | 24 | 48 | 72 |
| 421 | Ex. I | — | 3.75 | 4.2 | 3.3 | 0.94 | 0.24 | 0.09 |
| 422 | Ex. I | — | 2.40 | 3.5 | 1.7 | 0.76 | 0.15 | <0.09 |
| 423 | Ex. I | — | 2.6 | 4.3 | 2.5 | 0.86 | 0.28 | 0.14 |
| 424 | Ex. I | — | 2.5 | 3.8 | 2.0 | 0.70 | 0.18 | 0.12 |
| 426 | Ex. I | — | 3.9 | 4.9 | 2.5 | 0.82 | 0.22 | 0.11 |
| 428 | Ex. I | — | 2.5 | 3.5 | 2.6 | 0.96 | 0.44 | 0.22 |
| 429 | Ex. I | — | 3.6 | 3.0 | 2.0 | 0.86 | 0.11 | — |
| 431 | Ex. I | — | 3.0 | 3.2 | 2.6 | 0.74 | 0.26 | 0.16 |
| 432 | Ex. I | — | 4.2 | 2.8 | 2.3 | 1.2 | 0.32 | 0.13 |
| 434 | Ex. I | — | 2.4 | 2.5 | 1.7 | 0.74 | 0.15 | <0.09 |
| Av. | Ex. I | — | 3.1 | 3.6 | 2.3 | 0.9 | 0.24 | 0.1 |

### TABLE B
Oxytetracycline content in µg/ml in the serum of cows

| Cow | Composition | Hours after treatment | | | | | | | | | |
|---|---|---|---|---|---|---|---|---|---|---|---|
| | | 0 | 1/2 | 1 | 2 | 4 | 8 | 12 | 24 | 48 | 72 |
| 1 | Ex. I | — | 0.3 | 0.6 | 1.3 | 3.2 | 3.6 | 4.8 | 3.9 | 1.6 | 0.3 |
| 2 | Ex. I | — | 0.6 | 1.4 | 1.9 | 3.0 | 3.6 | 4.8 | 3.9 | 1.2 | 0.2 |
| 3 | Ex. I | — | 2.8 | 2.7 | 3.0 | 4.8 | 5.1 | 6.6 | 5.5 | 1.4 | 0.2 |
| 4 | Ex. I | — | 1.1 | 1.4 | 4.2 | 3.6 | 4.5 | 5.7 | 5.0 | 0.94 | 0.1 |
| 8 | Ex. I | — | 0.6 | 0.9 | 0.7 | 3.3 | 2.7 | 3.3 | 3.6 | 0.71 | 0.1 |
| Av. | Ex. I | — | 1.1 | 1.4 | 2.2 | 3.6 | 3.9 | 5.0 | 4.4 | 1.2 | 0.2 |

From these data and the graph one can see that the compositions according to the invention give an excellent oxytetracycline level in blood serum, which also after 48 h meets the required condition (at least 1 µg/ml).

Example V

A calf, 1 month old, was intravenously injected in the vena jugularis with 2.5 ml of the composition according to example III (500 mg oxytetracycline). The calf did not show any visible reaction, the breathing remained normal and the pulse (80 per min) remained unaltered.

A cow with a weight of 500 kg was slowly intravenously injected in the vena jugularis, with 10 ml of the composition according to example III.

The cow did not show any externally visible reaction; pulse and breathing remained unaltered.

Such an injection wherein coincidentally paravenous injection took place, provided a temporary oedema which disappeared quickly. From these tests it appears that the compositions are suitable for intravenous injection.

Example VI

To 9 cows of which 4 suffered from peritonitis and the others from bronchitis or bronchopnuemonia, the composition according to example I was administered intraperitoneally in an amount of 20 mg oxytetracycline/kg body weight (10 ml solution per 100 kg body weight).

The cows did not show any pain reaction after the injection or swelling.

The injection resulted in a clear improvement of the symptoms of disease in all cases.

**Claims**

1. An oxytetracycline composition which, on 100 parts by volume of the composition, comprises 10—30 parts by weight of oxytetracycline in the form of a complex with alkaline earth metal ions, 30—60 parts by volume of water, 2.5—10 parts by weight of polyvinylpyrrolidone, usual amounts of antioxidants, inorganic or organic bases in an amount sufficient to bring the pH of the composition at a value between 6.0 and 9.5, the balance being N-methylpyrrolidone.

2. Process for preparing an oxytetracycline composition, wherein a solution is formed of alkaline earth metal complexes of oxytetracycline in a mixture of water, water-miscible organic solvent and adjuvants with a pH between 6.0 and 9.5, characterized in that, 10—30 parts by weight of oxytetracycline in the form of the base, or an acid addition salt and sufficient alkaline earth metal ions to form a complex with the oxytetracycline 2.5—10 parts by weight of polyvinylpyrrolidone and usual amounts of antioxidant are incorporated into 30—60 parts by volume of water, the pH is adjusted at a value between 6.5 and 9.5 by adding an inorganic or organic base and the mixture is made up to a volume of 100 parts by volume with N-methylpyrrolidone.

3. Composition or process according to claim 1 or 2, characterized in that, 20—25 parts by weight of oxytetracycline in the form of the base or an acid addition salt are used.

4. Composition or process according to claim 1 or 2, characterized in that magnesium oxide is used as source of alkaline earth metal ions.

**Patentansprüche**

1. Oxytetracyclin-Zusammensetzung, welche auf 100 Volumenteilen der Zusammensetzung, 10—30 Gewichtsteile Oxytetracyclin in der Form eines Komplexes mit Erdalkalimetallionen, 30—60 Volumenteile Wasser, 2.5—10 Gewichtsteile Polyvinylpyrrolidon übliche Mengen von Antioxidanten und inorganischen oder organischen Basen in einer Menge ausreichend um den pH-Wert der Zusammensetzung zwischen 6.0 und 9.5 zu bringen, enthält, wobei der Rest N-Methylpyrrolidon ist.

2. Verfahren zur Herstellung einer Oxytetracyclin-Zusammensetzung, worin ein Lösung von Erdalkalimetallkomplexen von Oxytetracyclin gebildet wird, in einer Mischung aus Wasser, wassermischbarer organischer Lösungs- und Hilfsmittel mit einem pH-Wert zwischen 6.0 und 9.5 dadurch gekennzeichnet, dass 10—30 Gewichtsteile Oxytetracyclin in der Form der Base oder eines Säure-Additionssalzes und ausreichend Erdalkalimetallione zur Komplexbildung mit Oxytetracyclin, 2.5—10 Gewichtsteilen Polyvinylpyrrolidon und üblichen Mengen an Antioxidanten in 30—60 Gewichtsteilen Wasser, aufgenommen werden, der pH-Wert auf einen Wert zwischen 6.5 und 9.5 eingestellt wird unter der Hinzufügung einer inorganischen oder organischen Base und das Gemisch bis einem Volumen von 100 Volumenteilen mit N-Methylpyrrolidon ergänzt wird.

3. Zusammensetzung oder Verfahren nach Anspruch 1 oder 2, dadurch gekennzeichnet, dass 20—25 Gewichtsteile Oxytetracyclin in der Form einer Base oder eines Säure-Additions-salzes benutzt werden.

4. Zusammensetzung oder Verfahren nach Anspruch 1 oder 2, dadurch gekennzeichnet, dass Magnesiumoxid als Quelle der Erdalkalimetallionen verwendet wird.

**Revendications**

1. Composition d'oxytétracycline comportant, sur 100 parties en volume de la composition, 10—30 parties en poids d'oxytétracycline sous forme d'une complexe avec des ions alcalino-terreux, 30—60 parties en volume d'eau, 2,5—10 parties en poids de polyvinylpyrrolidone, des quantités usuelles d'antioxydants, et des bases organiques ou inorganiques dans une quantité qui suffit pour porter le pH de la composition à une valeur comprise entre 6,0 et 9,5, le reste étant la N-méthylpyrrolidone.

2. Procédé de préparation d'une composition d'oxytétracycline, selon lequel une solution est formé de complexes alcalinoterreux d'oxytétracycline dans un mélange d'eau, de solvant organique miscible à l'eau et d'additifs ayant un pH compris entre 6,0 et 9,5, caractérisé en ce que l'on incorpore dans 30—60 parties en volume d'eau, 10—30 parties en poids d'oxytétracycline sous forme de base, ou un sel d'addition d'acide et suffisamment d'ions alcalino-terreux pour former un complexe avec l'oxytétracycline, 2,5—10 parties en poids de polyvinylpyrrolidone et des quantités usuelles d'antioxydants, en ce que le pH est ajusté à une valeur comprise entre 6,5 et 9,5 en ajoutant une base organique ou inorganique, et en ce que le mélange est complété à un volume de 100 parties en volume par de la N-méthylpyrrolidone.

3. Composition ou procédé selon la revendication 1 ou 2, caractérisé en ce qu'l'on utilise 20—25 parties en poids d'oxytétracycline sous forme de base ou d'un sel d'addition d'acide.

4. Composition ou procédé selon la revendication 1 ou 2, caractérisé en ce que l'on utilise de l'oxyde de magnésium comme source d'ions alcalino-terreux..

comp. according to example IV, pigs

comp. according to example IV, cows

oxytetracycline concentration (µg/ml serum)

hours after administration

0 1 2   4   8   12        24              48                              72

0   1   2   3   4   5